(19) Europäisches Patentamt
European Patent Office
Office européen des brevets

(11) Publication number: **0 038 575**
**B1**

(12) # EUROPEAN PATENT SPECIFICATION

(45) Date of publication of patent specification: **09.03.83**

(51) Int. Cl.³: **C 07 C 121/70**, C 07 D 317/60, C 07 D 333/24, C 07 C 120/00

(21) Application number: **81104297.7**

(22) Date of filing: **30.07.79**

(60) Publication number of the earlier application in accordance with Art. 76 EPC: **0008867**

(54) **Cyanovinyl-cyclopropanecarboxylic acids and method of preparing these compounds.**

(30) Priority: **28.08.78 US 937359**
**28.08.78 US 937360**

(43) Date of publication of application:
**28.10.81 Bulletin 81/43**

(45) Publication of the grant of the patent
**09.03.83 Bulletin 83/10**

(84) Designated Contracting States:
**AT BE CH DE FR GB IT NL SE**

(56) References cited:
FR - A - 2 363 588

(73) Proprietor: **AMERICAN CYANAMID COMPANY**
**Berdan Avenue**
**Wayne New Jersey 06904 (US)**

(72) Inventor: **Brown, Dale Gordon**
**Stony Brook Road**
**Hopewell New Jersey (US)**

(74) Representative: **Allam, Peter Clerk et al,**
**LLOYD WISE, TREGEAR & CO. Norman House**
**105-109 Strand**
**London WC2R 0AE (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England

Cyanovinyl-cyclopropanecarboxylic acids and method of preparing these compounds

This invention relates to novel cyclopropanecarboxylic derivatives which are useful as intermediates in the preparation of cyanovinyl pyrethroid tickicidal agents, as disclosed in our European Patent Application No. 79301513.2 (Publication No. 0008867A) from which the present application is divided.

The novel compounds of the present invention are cyanovinyl-cyclopropanecarboxylic acids of the formula:

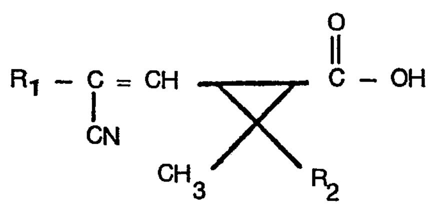

(IV)

wherein $R_1$ is

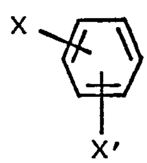 , 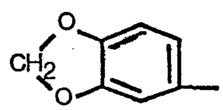 , 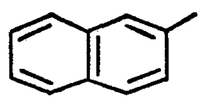 or 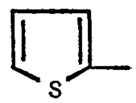 ;

$R_2$ is hydrogen or methyl; X and X′ are hydrogen, halogen, $C_1$—$C_3$ alkyl, $C_1$—$C_3$ alkoxy or haloalkyl $C_1$—$C_2$; with the proviso that X and X′ are not both hydrogen.

It will be recognised that the compounds of formula (IV) above may exist in a number of geometric and optical isomeric forms, and mixtures thereof. All such isomeric forms, and mixtures thereof, are within the scope of the present invention. Those isomers in which the carboxylic acid and the $\beta$-cyanostyryl functions are in the opposite sides of the cyclopropane ring are designated as *trans;* those in which they are on the same side are designated as *cis*. In each case, these may be separated into (+) and (—) optical isomers by appropriate methods, such as, for instance, the use of chiral bases. A further locale for geometrical isomerism is the $\beta$-cyanostyryl function itself. In this case the designation *Z* is given to those compounds in which the cyano and cyclopropyl substituents are on the same side of the carbon — carbon double bond, whereas the *E* isomer is that in which the aryl or heterocyclic and the cyclopropyl substituents are so situated.

The present invention also provides a method for the preparation of a compound of the formula:

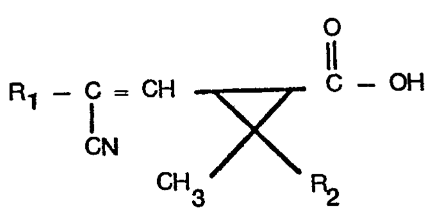

wherein $R_1$ is

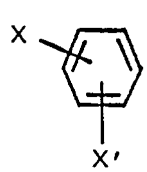 , 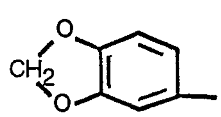 , 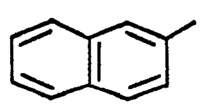 or 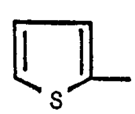 ;

$R_2$ is hydrogen or methyl; X and X′ are each hydrogen, halogen, $C_1$—$C_3$ alkyl, $C_1$—$C_3$ alkoxy or haloalkyl $C_1$—$C_2$; comprising

reacting a compound of formula (I):

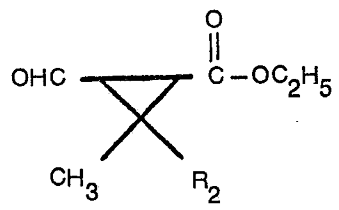

(I)

wherein $R_2$ is as hereinabove defined with a compound of formula (II) $R_1$—$CH_2$—CN in the presence of a $C_1$—$C_3$ alcohol and sodium ethoxide at ambient temperature for a period of time sufficient to essentially complete the condensation of the formyl group of the compound of formula (I) with the active methylene group of the compound of formula (II); wherein $R_1$ is as hereinabove defined.

The starting material for this process ie the compound of formula (I), may itself be prepared by oxidizing a compound of formula (II)

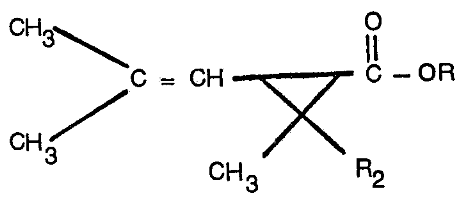

(II)

wherein R is a $C_1$—$C_3$ alkyl and $R_2$ is as hereinabove defined, with ozone at a temperature in the range −50° to −70°C in the presence of a $C_1$—$C_3$ alcohol.

The overall reaction may be represented by the following reaction scheme:

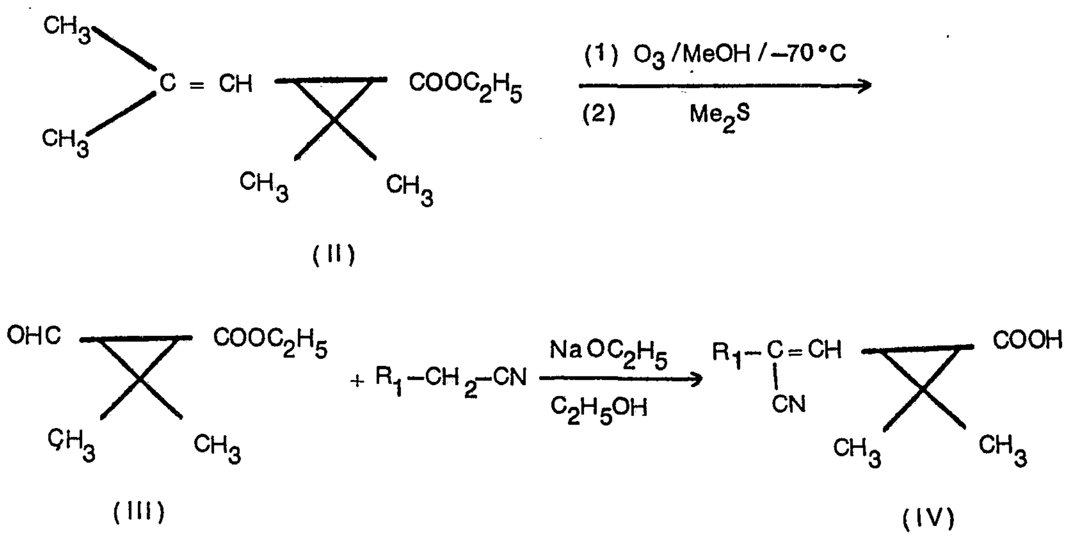


wherein in the above reaction sequence $R_1$ is as hereinbefore defined, $CH_3$— was chosen to represent $R_2$, and $C_2H_5$— was chosen to represent R.

Thus, ethyl chrysanthemate (II), wherein $R_2$ is methyl, is oxidized with ozone in a methanolic solution at −50° to −70°C to yield the corresponding 3-formyl-2,2-dimethylcyclopropanecarboxylic acid ethyl ester (III). Next, the thus obtained formyl compound is condensed in the presence of a base, such as sodium ethoxide in a lower alcohol with a compound of formula: $R_1$—$CH_2$—CN having an active methylene group to yield the desired cyclopropanecarboxylic acid of formula (IV).

The compounds of the present invention are useful as precursor compounds for the preparation of certain cyanovinyl pyrethroids having tickicidal activity, as more fully shown and described in our European Patent Application No. 79301513.2 (Publication No. 0008867A).

The following Examples illustrate the invention.

Example 1

Preparation of 3-formyl-2,2-dimethyl-cyclopropanecarboxylic acid, ethyl ester

A solution of ethyl chrysanthemate (156.0 g) in absolute methanol (1400 ml) is stirred, cooled to −65°C, and ozone bubbled in at a rate of 1.7 l/min. under 0.56 kg/cm$^2$ pressure (the ozone is generated with a Welsbach T—23 ozonator; voltage setting 120 VAC). During the reaction, the temperature range of the reaction mixture fluctuates between −65°C and −50°C.

The reaction is followed with gas chromatography [6′ × 1/4″ glass column, packed with 3% OV—1 on WHP; temperature 130°C; sample size 0.2$\lambda$; flow 45 ml/min He: FID detector] to measure the disappearance of ethyl chrysanthemate. The ozonolysis is stopped after about 6 to 7 hours when about 5% ethyl chrysanthemate is still present. Methyl sulfide (125 ml) is added over 20 minutes and the reaction mixture allowed to warm up to room temperature overnight with stirring. Next, the methanol is removed *in vacuo* at 50—55°C. The residual oil is diluted with ether, and the ether solution washed with water. The ether layer is dried over magnesium sulfate, filtered, and is then evaporated to yield 131.3 g of title product.

An nmr is run on the product to determine if any acetal which may have formed during the reaction (multiplet at 3.4) is present. If acetal is found in the product, 10% hydrochloric acid (85 ml) is added with stirring followed by the addition of sufficient amount of tetrahydrofuran to obtain a homogeneous solution. The solution is heated at 40—50°C for one hour. Water and ether are added, and the aqueous layer extracted with ether. The ether layers are combined, dried over magnesium sulfate and evaporated *in vacuo* to afford the title product.

Example 2

Preparation of *(Z)-cis* and *trans*-3-($\beta$-cyanostyryl)-2,2-dimethyl-cyclopropanecarboxylic acid

Phenylacetonitrile is added to a stirred solution of sodium ethoxide in absolute ethanol (prepared from 1.1 g sodium and 100 ml of ethanol). Next, a solution of 3-formyl-2,2-dimethyl-cyclopropanecarboxylic acid ethyl ester (8.5 g) in absolute ethanol (15 ml) is added over 5 minutes. The reaction mixture is stirred for 12 hours at room temperature and then heated on a steam bath for 0.5 hour. The alcohol is removed *in vacuo*, and the residue washed with water (250 ml) and ether (150 ml). The aqueous layer is acidified with concentrated hydrochloric acid. The resulting cream colored slurry is filtered and the isolated product dried. The product is dissolved in ether and precipitated with hexane to afford 6.1 g of white crystals, m.p. 186—188°C.

By the above procedure, a number of cyclopropanecarboxylic acids are prepared. These are listed in the Table below, together with their melting points.

TABLE

Substituted Cyclopropanecarboxylic Acids

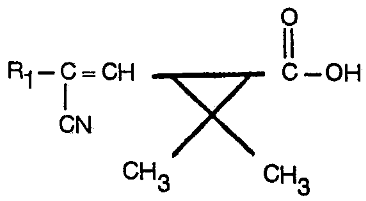

| $R_1$ | m.p. °C. |
|---|---|
| 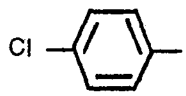 | 190–208 |
| 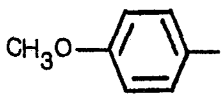 | 188–198 |
| 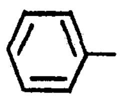 | 120–142 |
| 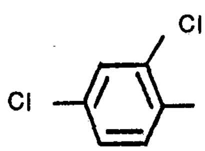 | 130–145 |
| 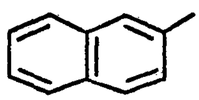 | 217–218 |
| 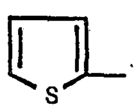 | 140–143 |
| 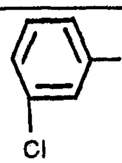 | 125–155 |
| 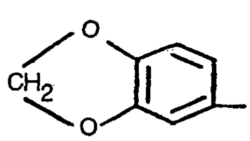 | 160–180 |

TABLE (Continued)

| $R_1$ | m.p. °C. |
|---|---|
| 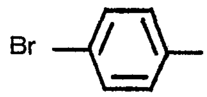 | 179–195 |
| 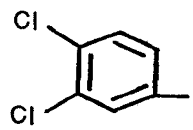 | 195–202 |
| 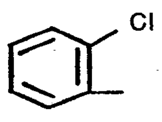 | * |
| 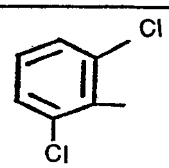 | 103–112 |
| 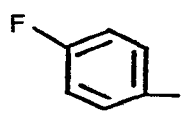 | 150–164 |
| 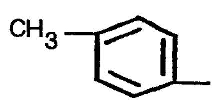 | 164–176 |
| 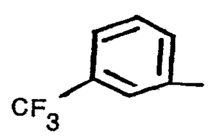 | 117–123 |

* = Gum – NMR TMS – $CDCl_3$ doublet – 6.30 δ doublet 7.05 δ

## Example 3

Preparation of 2-Formyl-3-methylcyclopropanecarboxylic acid ethyl ester

Crotonaldehyde (14.0 g; 0.2 mole) is added dropwise to a gently refluxing solution of ethyl tetrahydrothiophenium carbox-methylide (34.0 g; 0.2 mole) in dry acetone (200 ml). Heating at reflux is continued for about 15 minutes after the addition is completed, then the reaction mixture is concentrated on a rotary evaporator at 50°C. Distillation of this concentrate at reduced pressure affords 15.32 g (49.1%) of title product, a pale yellow oil b.p. 0.1 mm, 44—45°C. The structure is confirmed by IR and NMR.

## Example 4

Preparation of *(Z)-cis* and *trans*-2-(β-cyanostyryl)-3-methylcyclopropanecarboxylic acid

A solution of sodium ethoxide is prepared by adding sodium spheres (1.15 g, 0.05 mole) to absolute ethanol (100 ml) with stirring until a solution is obtained. Phenylacetonitrile (5.86 g, 0.05

mole) and 2-formyl-3- methylcyclopropanecarboxylic acid methyl ester (7.81 g, 0.05 mole) are added to the above solution at room temperature. The resultant clear solution is stirred for 18 hours at room temperature, then heated at reflux for about 0.5 hours. The reaction mixture is then cooled, evaporated *in vacuo*, and the residue dissolved in water. The aqueous solution is extracted (3X) with ether and is then acidified with concentrated hydrochloric acid. A brown solid precipitates and is isolated by filtration and dried to afford 8.78 g (77.3%) of a tacky brown solid. NMR data supports the presence of both isomers (the olefin proton of the two isomers appearing as doublets at 6.17 ppm and 6.96 ppm).

**Claims for the Contracting States: BE CH DE FR GB IT NL SE**

1. A compound of the formula:

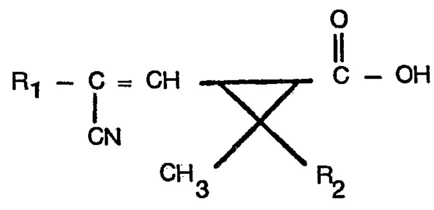

wherein $R_1$ is

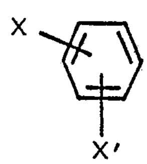, 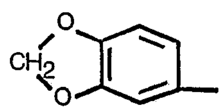, 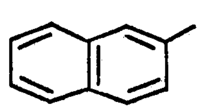, or 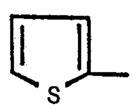 ;

$R_2$ is hydrogen or methyl; X and X′ are hydrogen, halogen, $C_1$—$C_3$ alkyl, $C_1$—$C_3$ alkoxy or haloalkyl $C_1$—$C_2$; provided that X and X′ are not both hydrogen.

2. A compound according to Claim 1, wherein $R_1$ is

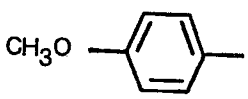, 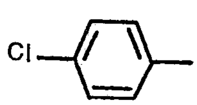, 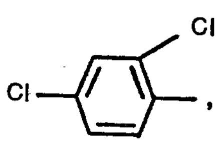, 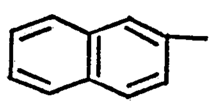 or 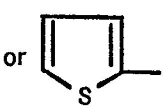 ;

and $R_2$ is methyl.

3. The compound according to Claim 2: (*Z*)-*cis* and *trans*-3-(β-cyano-*p*-methoxystyryl)-2,2-dimethyl-cyclopropanecarboxylic acid; (*Z*)-*cis* and *trans*-3-(*p*-chloro-β-cyanostyryl)-2,2-dimethyl-cyclopropanecarboxylic acid; (*Z*)-*cis* and *trans*-3-(*o*-chloro-β-cyanostyryl)-2,2-dimethylcyclopropanecarboxylic acid; (*Z*)-*cis* and *trans*-3-(*m*-chloro-β-cyanostyryl)-2,2-dimethyl-cyclopropanecarboxylic acid; (*Z*)-*cis* and *trans*-3-(2,4-dichloro-β-cyanostyryl)-2,2-dimethyl-cyclopropanecarboxylic acid; (*Z*)-*cis* and *trans*-3-(2,6-dichloro-β-cyanostyryl)-2,2-dimethyl-cyclopropanecarboxylic acid; (*Z*)-*cis* and *trans*-3-(3,4-dichloro-β-cyanostyryl)-2,2-dimethyl-cyclopropanecarboxylic acid; (*Z*)-*cis* and *trans*-3-(p-bromo-β-cyanostyryl)-2,2-dimethyl-cyclopropanecarboxylic acid; and (*Z*)-*cis* and *trans*-3-(β-cyano-*p*-fluorostyryl)-2,2-dimethyl-cyclopropanecarboxylic acid.

4. A method for the preparation of a compound of the formula:

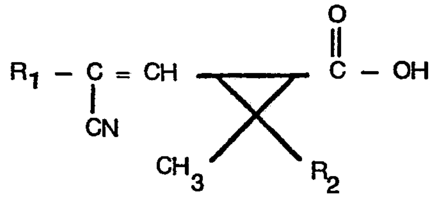

wherein $R_1$ is

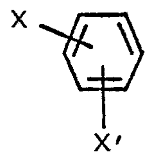, 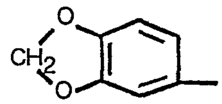, 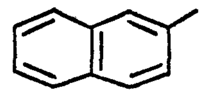 or 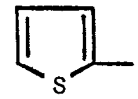;

$R_2$ is hydrogen or methyl; X and X′ are each hydrogen, halogen, $C_1$—$C_3$ alkyl, $C_1$—$C_3$ alkoxy or haloalkyl $C_1$—$C_2$; comprising:

reacting a compound of formula (I):

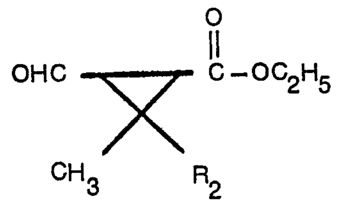 (I)

wherein $R_2$ is as hereinabove defined with a compound of formula (II) $R_1$—$CH_2$—CN in the presence of a $C_1$—$C_3$ alcohol and sodium ethoxide at ambient temperature for a period of time sufficient to essentially complete the condensation of the formyl group of the compound of formula (I) with the active methylene group of the compound of formula (II); wherein $R_1$ is as hereinabove defined.

5. A method according to Claim 4, wherein $R_1$ is

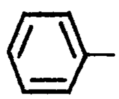, 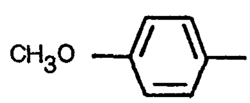, 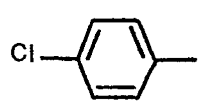, 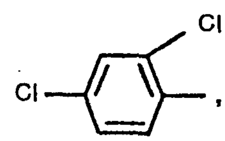,

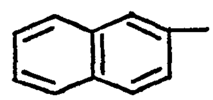 or 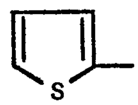;

and $R_2$ is methyl.

**Claims for the Contracting State: AT**

1. A method for the preparation of a compound of the formula:

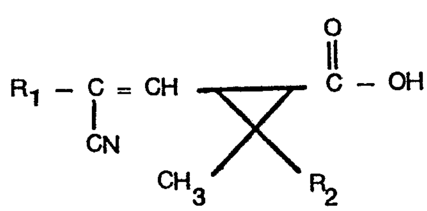

wherein $R_1$ is

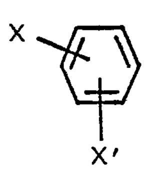, 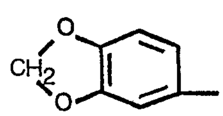, 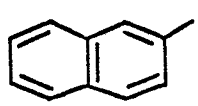 or 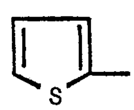;

$R_2$ is hydrogen or methyl; X and X′ are each hydrogen, halogen, $C_1$—$C_3$ alkyl, $C_1$—$C_3$ alkoxy or haloalkyl $C_1$—$C_2$; comprising
reacting a compound of formula (I):

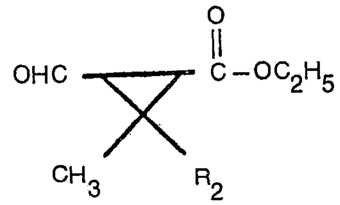

(I)

wherein $R_2$ is as hereinabove defined with a compound of formula (II) $R_1$—$CH_2$—CN in the presence of a $C_1$—$C_3$ alcohol and sodium ethoxide at ambient temperature for a period of time sufficient to essentially complete the condensation of the formyl group of the compound of formula (I) with the active methylene group of the compound of formula (II); wherein $R_1$ is as hereinabove defined.

2. A method according to Claim 1, wherein $R_1$ is

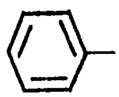 , 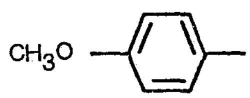 , 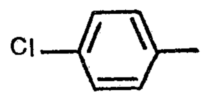 , 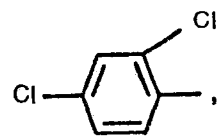 ,

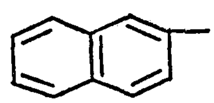 or 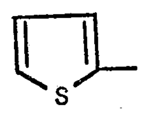 ;

and $R_2$ is methyl.

**Patentansprüche für die Vertragsstaaten: BE CH DE FR GB IT NL SE**

1. Verbindung der Formel

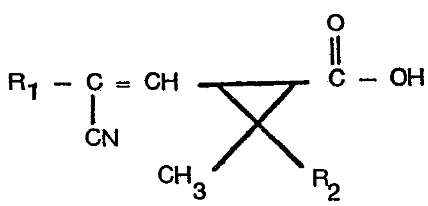

wobei $R_1$ für

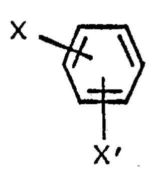 , 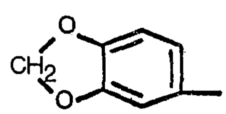 , 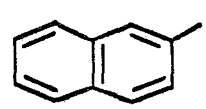 oder 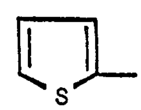 ;

steht; $R_2$ Wasserstoff oder Methyl bedeutet; X und X′ für Wasserstoff, Halogen, $C_{1-3}$-Alkyl, $C_{1-3}$-Alkoxy oder Halogenalkyl-$C_{1-2}$ stehen, mit der Maßgabe, daß X und X′ nicht beide Wasserstoff bedeuten.

2. Verbindung nach Anspruch 1, dadurch gekennzeichnet, daß $R_1$ für

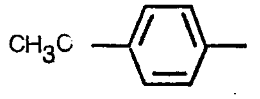 , 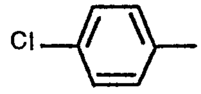 , 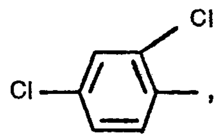 ,

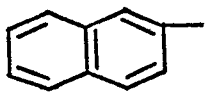 oder 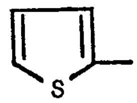 ;

steht und $R_2$ Methyl bedeutet.

3. Die Verbindungen nach Anspruch 2, nämlich (Z)-cis- und -trans-3-(β-Cyano-p-methoxystyryl)-2,2-dimethyl-cyclopropancarbonsäure; (Z)-cis- und -trans-3-(p-Chlor-β-cyanostyryl)-2,2-dimethyl-cyclopropancarbonsäure; (Z)-cis- und -trans-3-(o-Chlor-β-cyanostyryl)-2,2-dimethyl-cyclopropancarbonsäure; (Z)-cis- und -trans-3-(m-Chlor-β-cyanostyryl)-2,2-dimethyl-cyclopropancarbonsäure; (Z)-cis- und -trans-3-(2,4-Dichlor-β-cyanostyryl)-2,2-dimethyl-cyclopropancarbonsäure; (Z)-cis- und -trans-3-(2,6-Dichlor-β-cyanostyryl)-2,2-dimethyl-cyclopropancarbonsäure; (Z)-cis- und -trans-3-(3,4-Dichlor-β-cyanostyryl)-2,2-dimethyl-cyclopropancarbonsäure; (Z)-cis- und -trans-3-(p-Brom-β-cyanostyryl)-2,2-dimethyl-cyclopropancarbonsäure; und (Z)-cis- und -trans-3-(β-Cyano-p-fluorostyryl)-2,2-dimethyl-cyclopropancarbonsäure.

4. Verfahren zur Herstellung einer Verbindung der Formel

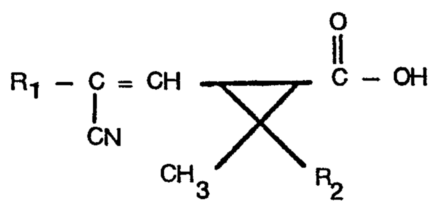

wobei $R_1$ für

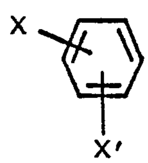 , 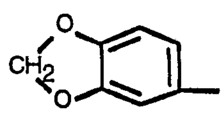 , 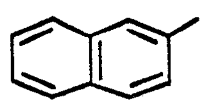 oder 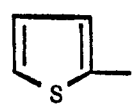 ;

steht; $R_2$ Wasserstoff oder Methyl bedeutet; X und X′ jeweils Wasserstoff, Halogen, $C_{1-3}$-Alkyl, $C_{1-3}$-Alkoxy oder Halogenalkyl-$C_{1-2}$ bedeuten;
dadurch gekennzeichnet, daß man eine Verbindung der Formel (I)

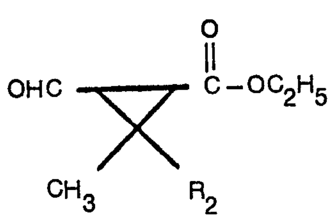

(I)

wobei $R_2$ die oben angegebene Bedeutung hat, mit einer Verbindung der Formel (II) $R_1$—$CH_2$—CN umsetzt, und zwar in Gegenwart eines $C_{1-3}$-Alkohols und Natriumäthoxid bei Umgebungstemperatur während einer Zeitspanne, die ausreicht, um die Kondensation der Formylgruppe der Verbindung der Formel (I) mit der aktiven Methylengruppe der Verbindung der Formel (II), wobei $R_1$ die oben angegebene Bedeutung hat, im wesentlichen vollständig zu bewirken.

5. Verfahren nach Anspruch 4, dadurch gekennzeichnet, daß $R_1$ für

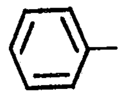 , 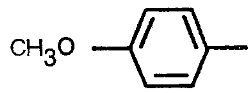 , 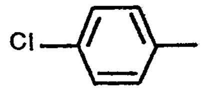 , 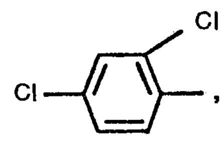 ,

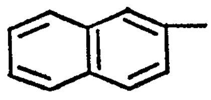 oder 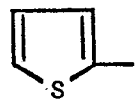 ;

steht und $R_2$ Methyl bedeutet.

**Patentansprüche für den Vertragsstaat: AT**

1. Verfahren zur Herstellung einer Verbindung der Formel

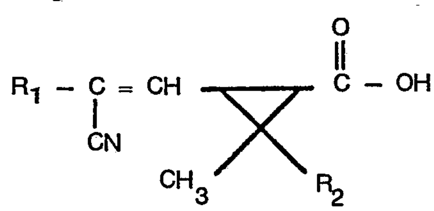 (IV)

wobei $R_1$ für

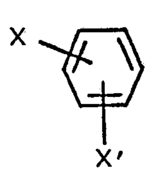 , 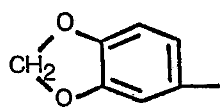 , 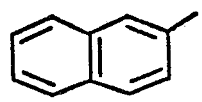 oder 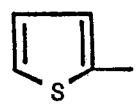 ;

steht; $R_2$ Wasserstoff oder Methyl bedeutet; X und X′ jeweils Wasserstoff, Halogen, $C_{1-3}$-Alkyl, $C_{1-3}$-Alkoxy oder Halogenalkyl-$C_{1-2}$ bedeuten;
dadurch gekennzeichnet, daß man eine Verbindung der Formel (I)

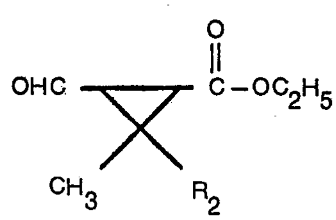 (I)

wobei $R_2$ die oben angegebene Bedeutung hat, mit einer Verbindung der Formel (II) $R_1—CH_2—CN$ umsetzt, und zwar in Gegenwart eines $C_{1-3}$-Alkohols und Natriumäthoxid bei Umgebungstemperatur während einer Zeitspanne, die ausreicht, um die Kondensation der Formylgruppe der Verbindung der Formel (I) mit der aktiven Methylengruppe der Verbindung der Formel (II), wobei $R_1$ die oben angegebene Bedeutung hat, im wesentlichen vollständig zu bewirken.

2. Verfahren nach Anspruch 1, dadurch gekennzeichnet, daß $R_1$ für

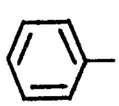 , 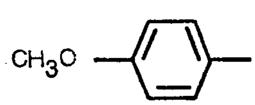 , 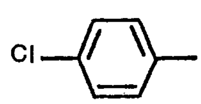 , 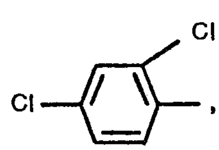 ,

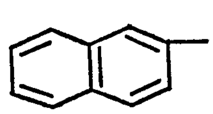 oder 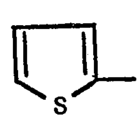 ;

steht und $R_2$ Methyl bedeutet.

**Revendications pour les Etats contractants: BE CH DE FR GB IT NL SE**

1. Composé de formule:

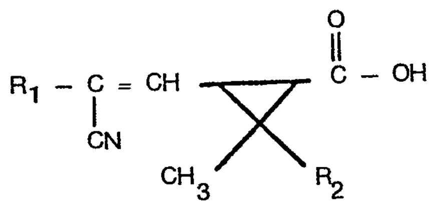

(IV)

dans laquelle $R_1$ est

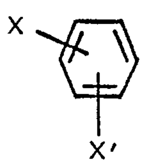 , 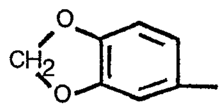 , 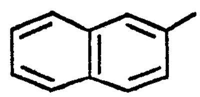 ou 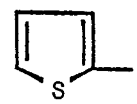 ;

$R_2$ est l'hydrogène ou un groupe méthyle; X et X' sont l'hydrogène, un groupe alkyle en $C_1$—$C_3$ un groupe alcoxy en $C_1$—$C_3$ ou un groupe halogénoalkyle en $C_1$—$C_2$; sous réserve que X et X' ne soient pas tous deux l'hydrogène.

2. Composé selon la revendication 1 où $R_1$ est

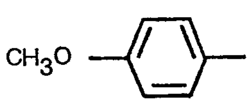 , 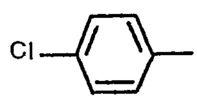 , 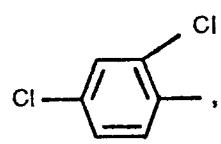 ,

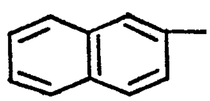 ou 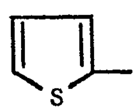 ;

et $R_2$ est un groupe méthyle.

3. Composés selon la revendication 2 qui sont l'acide (*Z*)-*cis* et *trans*-3-(β-cyano-p-méthoxy-styryl)-2,2-diméthyl-cyclopropanecarboxylique; l'acide (*Z*)-*cis* et *trans*-3-(p-chloro-β-cyanostyryl)-2,2-diméthyl-cyclopropanecarboxylique; l'acide (*Z*)-*cis* et *trans*-3-(p-chloro-β-cyanostyryl)-2,2-diméthyl-cyclopropanecarboxylique; l'acide (*Z*)-*cis* et *trans*-3-(m-chloro-β-cyanostyryl)-2,2-diméthyl-cyclopropanecarboxylique; l'acide (*Z*)-*cis* et *trans*-3-(2,4-dichloro-β-cyanostyryl)-2,2-diméthyl-cyclopropanecarboxylique; l'acide (*Z*)-*cis* et *trans*-3-(2,6-dichloro-β-cyanostyryl)-2,2-diméthyl-cyclopropanecarboxylique; l'acide (*Z*)-*cis* et *trans*-3-(3,4-dichloro-β-cyanostyryl)-2,2-diméthyl-cyclopropanecarboxylique; l'acide (*Z*)-*cis* et *trans*-3-(p-bromo-β-cyanostyryl)-2,2-diméthyl-cyclopropanecarboxylique; et l'acide (*Z*)-*cis* et *trans*-3-(β-cyano-p-fluorostyryl)-2,2-diméthyl-cyclopropanecarboxylique.

4. Procédé de préparation d'un composé de formule:

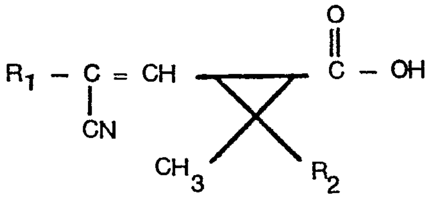

où $R_1$ est

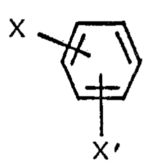 , 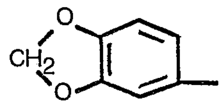 , 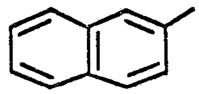 ou 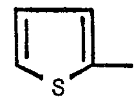 ;

$R_2$ est l'hydrogène ou un groupe méthyle; X et X' sont chacun l'hydrogène, un halogène, un groupe alkyle en $C_1$—$C_3$, un groupe alcoxy en $C_1$—$C_3$ ou un groupe halogénoalkyle en $C_1$—$C_2$; qui comprend la réaction d'un composé de formule (I):

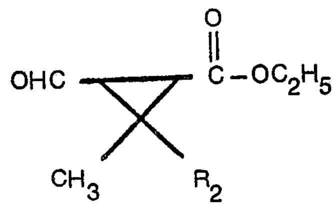 (I)

où $R_2$ a la même définition que ci-dessus avec un composé de formule (II) $R_1$—$CH_2CN$ en présence d'un alcool en $C_1$—$C_3$ et d'éthylate de sodium à la température ambiante pendant une durée suffisante pour achever essentiellement la condensation du groupe formyle du composé de formule (I) avec le groupe méthylène actif du composé de formule (II) où $R_1$ a la même définition que précédemment.

5. Procédé selon la revendication 4, dans lequel $R_1$ est

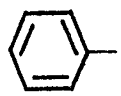 , 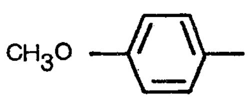 , 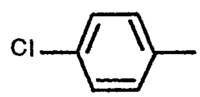 , 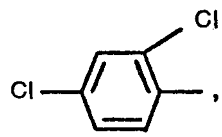 ,

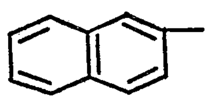 ou 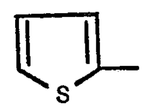 ;

et $R_2$ est un groupe méthyle.

**Revendications pour l'État contractant: AT**

1. Procédé de préparation d'un composé de formule:

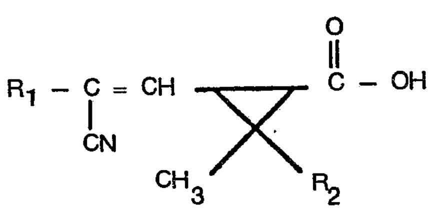

où $R_1$ est

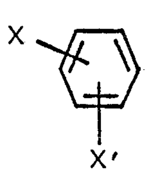 , 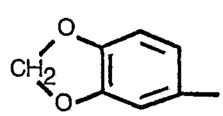 , 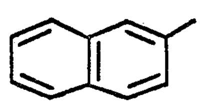 ou 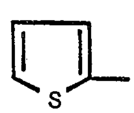 ;

$R_2$ est l'hydrogène ou un groupe méthyle; X et X' sont chacun l'hydrogène, un halogène, un groupe alkyle en $C_1$—$C_3$, un groupe alcoxy en $C_1$—$C_3$ ou un groupe halogénoalkyle en $C_1$—$C_2$; qui comprend la réaction d'un composé de formule (I):

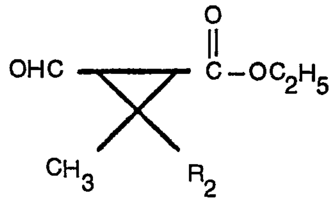

(I)

où $R_2$ a la même définition que ci-dessus avec un composé de formule (II) $R_1$—$CH_2$—CN en présence d'un alcool en $C_1$—$C_3$ et d'éthylate de sodium à la température ambiante pendant une durée suffisante pour achever essentiellement la condensation du groupe formyle du composé de formule (I) avec le groupe méthylène actif du composé de formule (II) où $R_1$ a la même définition que précédemment.

2. Procédé selon la revendication 1, dans lequel $R_1$ est

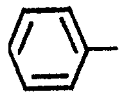 , 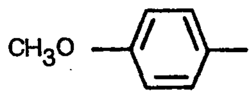 , 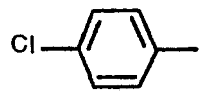 , 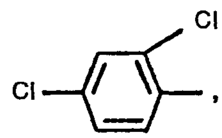 ,

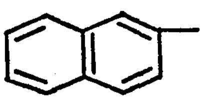 ou 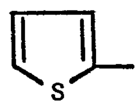 ;

et $R_2$ est un groupe méthyle.